# EUROPEAN PATENT SPECIFICATION

(11) **EP 1 569 651 B1**
(45) Date of publication and mention of the grant of the patent: **30.07.2008**
(21) Application number: 03779899.8
(22) Date of filing: 12.11.2003
(51) Int. Cl.: A61K 31/495, A61K 9/20, A61K 31/135

(54) **TABLET COMPRISING EFLETIRIZINE AND PSEUDOEPHEDRINE**
TABLETTE ENTHALTEND EFLETIRIZIN UND PSEUDOEPHEDRIN
COMPRIME A BASE D'EFLETIRIZINE ET DE PSEUDOEPHEDRINE

(30) Priority: 06.12.2002 EP 02080127
(43) Date of publication of application: 07.09.2005
(73) Proprietor: UCB FARCHIM S.A., CH-1630 Bulle (CH)
(72) Inventor: BERWAER, Monique, B-6120 Ham-Sur-Heure-Nalinnes (BE); GUICHAUX, Anthony, B-1180 Bruxelles (CH); CUYPERS, Serge, B-1470 Baisy-Thy (BE); DELEERS, Michel, B-1630 Linkebeek (BE); FANARA, Domenico, B-4520 Wanze (BE)
(74) Representative: Lechien, Monique
(86) International application number: PCT/EP2003/012627
(87) International publication number: WO 2004/052367

(56) References cited:
- WO-A-98/30208
- WO-A-98/41194
- WO-A-99/32125
- US-B1- 6 171 618
- US-B1- 6 469 009

## Description

The present invention concerns a tablet comprising two distinct segments. More particularly the invention relates to combinations of two pharmaceutical substances and methods of treatment of allergic disorders.

2-{2-[4-[bis(4-fluorophenyl)methyl]-1-piperazinyl]ethoxy}acetic acid or efletirizine, in the form of its dihydrochloride salt has the following formula:

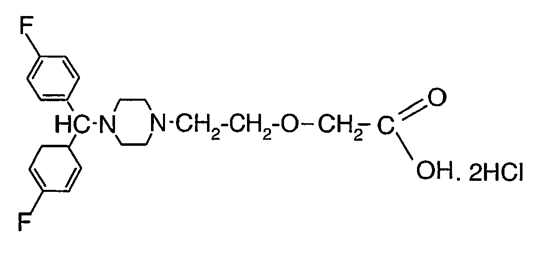

Efletirizine is encompassed within the general formula of European Patent No. 0 058 146 and may be prepared according to the general process described in this patent. Said process for the synthesis of 2-{2-[4-(diphenylmethyl)-1-piperazinyl]ethoxy}acetic acid derivatives comprises reacting a 1-(diphenylinethyl) piperazine derivative with methyl(2-chloroethoxy)acetate or 2-(2-chloroethoxy) acetamide to form a methyl 2-{2-[4-(diphenylmethyl)-l-piperazinyl]ethoxy}-acetate or a 2- {2-[4-(diphenylmethyl)-1-piperazinyl] ethoxy} acetamide, respectively. Thus the formed methyl ester or acetamide is then subjected to basic hydrolysis followed by acidification and isolation of the free carboxylic acid which is then transformed into its dihydrochloride.

European Patent N° 1 034 171 describes two pseudo-polymorphic forms of efletirizine.

Efletirizine has been found to possess excellent antihistaminic properties. It belongs to the pharmacological class of second generation histamine H₁-receptor antagonists and shows in vitro high affinity and selectivity for H₁-receptors. It is useful as an antiallergic, antihistaminic, bronchodilator and antispasmodic agent, and also for the treatment of allergic rhinitis and rhino-conjunctivitis.

On the other hand, a compound pseudoephedrine, is well known as sympathomimetic drug recognised as safe therapeutic agents effective in the relief of nasal congestion.

It is well known to those skilled in the art that combinations of pharmaceutical substances should always be handled with care because they are very susceptible of inducing unpredictable adverse effects in humans. In some cases, they also induce an efficiency of the treatment which is lower than that of each pharmaceutical substance taken alone.

In the treatment of allergic disorders such as for example a pollen associated allergic rhino-conjunctivitis, care should be taken, when combining an antihistaminic and a decongestant, not only to increase the overall efficiency of the treatment, i.e. the percentage of days during the whole treatment period, when the symptoms of sneezing, rhinorrhea, nasal obstruction, lacrimation, nasal and ocular pruritus are absent or at the most mild, but also to avoid possible adverse effects like insomnia and headache.

Several patent applications already disclosed binary and/or ternary combinations of pharmaceutical substances in specific amounts in view of treating various disorders in humans. In particular United Kingdom Patent 2 311 940 and European patent application 0 811 374 disclose a pharmaceutical composition comprising cetirizine and pseudoephedrine; US Patent 6,171,618 discloses a dosage form containing cetirizine as an immediate release component and pseudoephedrine as a controlled release component, a portion of the pseudoephedrine can be incorporated as an immediate release component.

In a more particular way, the international patent application WO 98/41194 discloses a pharmaceutical composition which can be administered orally, allowing the immediate release of a first active substance and the prolonged release of the same or of a second active substance, comprising
A. at least one layer comprising an active substance and excipients which allow immediate release of the said active substance after administration, and
B. at least one second layer which allows the controlled release of the same or of a second active substance, this layer being a pharmaceutical composition comprising between 5 and 60% by weight, relative to the total weight of the composition, of at least one excipient, selected from inert matrices, hydrophilic matrices, lipid matrices, mixtures of inert matrices and of lipid matrices, mixtures of hydrophilic matrices and of inert matrices; and between 5 and 50% by weight, relative to the total weight of the composition, of at least one alkalinizing agent soluble in an aqueous phase under physiological pH conditions.

Due to the presence of the alkalinizing agent, this composition has demonstrated a good stability profile.

It has now surprisingly been found that such a pharmaceutical composition can be prepared by adding less than 5 % of alkalinizing agent or in the absence of alkalinizing agent.

Despite the fact that a lower amount of alkalinizing agent has been added, the tablet of the invention has also demonstrated a good stability profile.

Thus an objective of the present invention is to provide a useful combination of pharmaceutical substances for treating various disorders in humans, said combination being able to increase the efficiency of said treatment over the efficiency of each substance alone, while avoiding adverse effects during the said treatment.

Another objective of the present invention is to provide such a useful combination of pharmaceutical substances when the treatment in question is a therapy such as needed for rhinitis, cold, flu, cold-like and flu-like symptoms.

The present invention describes a method of treating a disorder selected from rhinitis, cold, flu, cold-like and flu-like symptoms in a human, which comprises administering to a human in need of such therapy, a tablet comprising an effective amount of pseudoephedrine, an individual optical isomer or a pharmaceutically acceptable salt thereof and an effective amount of efletirizine or a pharmaceutically acceptable salt thereof.

The term "a method for treating a disorder selected from rhinitis, cold, flu, cold-like and flu-like symptoms in a human" as used herein means providing relief from the symptoms of sneezing, rhinorrhea, nasal obstruction, nasal and ocular pruritus, lacrymation, and the like.

The present invention provides a bi-layer tablet comprising at least two distinct segments, one segment of which comprises as active ingredient predominantly efletirizine and a second segment of which comprises as active ingredient predominantly pseudoephedrine, wherein the tablet is substantially free of impurities formed by reaction of efletirizine with pseudoephedrine, and with the proviso that the tablet comprises less than 5 % by weight, relative to the total weight of the pseudoephedrine segment, of an alkalinizing agent.

Further embodiments of the invention are defined by the dependent claims.

The term "pharmaceutically acceptable salts" as used herein with respect to efletirizine means not only their addition salts with non-toxic organic and inorganic acids, such as acetic, citric, succinic, ascorbic, hydrochloric, hydrobromic, sulfuric, and phosphoric acids and the like, but also their metal salts (for example sodium or potassium salts), ammonium salts, amine salts and aminoacid salts.

The term "pharmaceutically acceptable salt" as used herein with respect to pseudoephedrine means namely its hydrochloride and sulfate and equivalent non-toxic salts.

The term "individual optical isomer" as used herein means, when the molecule has a centre of asymmetry, the levorotatory and the dextrorotatory enantiomers thereof. As is well known in the art, purification of such enantiomers is a rather difficult process depending upon the selected way of preparation of the compound and the optical purity of the starting material. Therefore the term "individual optical isomer "as used herein means that the said compound comprises at least 90%, preferably at least 95%, by weight of the said individual (either dextro- or levorotatory) optical isomer and at most 10%, preferably at most 5%, by weight of the other individual (respectively levo- or dextrorotatory) optical isomer. Additionally, each individual optical isomer can be prepared from the racemic mixture by enzymatic biocatalytic resolution, such as disclosed in U.S. Patents No. 4,800,162 and 5,057,427.

The preferred compounds for efletirizine are the acid and its dihydrochloride salt.

In the present application the term "pseudoephedrine", used herein means pseudoephedrine itself, an individual optical isomer or a pharmaceutically acceptable salt thereof.

In the present application the term "efletirizine" means efletirizine itself (2-{2-[4-[bis(4-fluorophenyl)methyl)-1-piperazinyl]ethoxy}acetic acid), or a pharmaceutically acceptable salt thereof.

In a particular embodiment, the present invention concerns a tablet comprising at least two distinct segments, one segment of which comprises as active ingredient predominantly efletirizine and a second segment of which comprises as active ingredient predominantly pseudoephedrine, said segments being composed and formed in such a way that the resulting tablet is substantially free of impurities formed by reaction of efletirizine with pseudoephedrine and with the proviso that the tablet comprises less than 5 % by weight, relative to the total weight of the pseudoephedrine segment, of an alkalinizing agent. Indeed, it was demonstrated that if efletirizine and pseudoephedrine were formulated together in the same segment, a degradation of efletirizine appeared due to a chemical reaction with pseudoephedrine.

In a second embodiment, the invention concerns a tablet comprising at least two distinct segments one segment of which comprises as active ingredient predominantly efletirizine and a second segment of which comprises as active ingredient predominantly pseudoephedrine, said segments being composed and formed in such a way that the pharmacokinetic profiles of the efletirizine and pseudoephedrine are substantially the same as in a dosage form containing each as sole active ingredient in the same amount.

By the term "segment" we understand a discrete volume of a pharmaceutical composition containing an active drug and one or more pharmaceutically acceptable excipients. A segment of a tablet may form, for example, a layer of a multilayer tablet (i.e. a layer of a bilayer tablet) or a core of a tablet or a coating fully or partially covering a core of a tablet. A segment may also be a particle fully or partially covered by a coating or a coating fully or partially covering a particle.

By "substantially free" we understand less than 5 %, preferably less than 3 % by weight. More preferably we understand less than 0.5 %, further more less than 0.2 % by weight.

Preferably, in the tablet according to the invention, the pseudoephedrine segment is substantially free of efletirizine, by which is meant less than 5 %, preferably less than 3 %, more preferably less than 0.5 % of the efletirizine segment content in the pseudoephedrine segment. Preferably, in the tablet according to the invention, the efletirizine segment is substantially free of pseudoephedrine, by which is meant less than 5 %, preferably less than 3 %, more preferably less than 0.5 % of the pseudoephedrine segment content in the efletirizine segment.

In another embodiment of the invention the tablet further comprises a barrier segment wherein said barrier segment separates the efletirizine segment and the pseudoephedrine segment. The barrier segment comprises materials known to persons skilled in the art.

In another embodiment of the invention, the pseudoephedrine segment comprises less than 5 % by weight, relative to the total weight of the pseudoephedrine segment, of an alkalinizing agent.

The alkalinizing agent which can be used according to the present invention should preferably be soluble in the aqueous phase under physiological pH conditions. The alkalinizing agent may be chosen from alkali or alkaline-earth metal hydroxides, carbonates, bicarbonates and phosphates, sodium borate as well as basic salts of organic acids (example: sodium citrate). On the other hand, salts not soluble in water under physiological pH conditions, such as dibasic calcium phosphate, are not suitable according to the present invention.

In another embodiment of the invention, the tablet comprises a plurality of pseudoephedrine segments.

Preferably the pseudoephedrine segment of the tablet contains inert pharmaceutical excipients in an amount of 0.75 to 4.5 times that of the pseudoephedrine itself by weight, and more preferably of 1 to 3 times.

Preferably the efletirizine segment of the tablet contains inert pharmaceutical excipients in an amount of 5 to 30 times that of the efletirizine itself by weight, and more preferably of 10 to 20 times.

Preferably the ratio of the total amount of inert pharmaceutical excipients present to the total aggregate amount of all active ingredients is between 1.2 and 6 by weight. The best results have been obtained with a ratio of about 3. In a preferred b.i.d. (b.i.d. = twice a day) tablet according to the invention the weight ratio of pseudoephedrine to efletirizine is between 2 and 40. The best results have been obtained with a ratio of about 12.

In a more preferred b.i.d. tablet the pseudoephedrine segment comprises about 108 to 160 mg, preferably 90 to 150 mg and more preferably 120 mg of pseudoephedrine and the effetirizine segment comprises about 3 to 25 mg and preferably 15 mg of efletirizine.

In that case, according to the invention, the interfacial surface area of the pseudoephedrine segment and efletirizine segment is less than 180 mm², and preferably from about 20 to about 150 mm² . By interfacial area we understand the calculated contact surface between the two segments what ever the type of tablet (round, oblong, squared, caplet, ...) or the type of contact could be.

In a preferred embodiment of the invention the pseudoephedrine segment is a slow release formulation. By "slow release", we understand a release of 10 to 60 % in 1 hour, and greater than 70 % in 6 hours, or 40 to 80 % in 2 hours, and greater than 70 % in 6 hours in 500 ml water (HCl 0.1N) in USP apparatus 1 (37 °C, 100 RPM).

In a preferred embodiment of the invention the efletirizine is in immediate release form. By "immediate release" we understand a release of more than 70 % in 30 minutes, in 500 ml water (HCl 0.1N) in USP apparatus 1 (37 °C, 100 RPM).

The b.i.d tablet weight is between 200 to 800 mg, and preferably between 300 and 600 mg.

In a preferred once a day tablet the pseudoephedrine segment comprises about 90 to 265 mg of pseudoephedrine and the efletirizine segment comprises about 15 to 70 mg of efletirizine and parameters of this tablet (for example interfacial area between segments, weight limits of the tablet, ...) have to be adapted by persons skilled in the art.

Preferably the tablet according to the invention comprises an amount of efletirizine which when dosed to a human subject gives a efletirizine area under the efletirizine plasma concentration versus time curve which is between 80 % and 125 % of the area under the efletirizine plasma concentration versus time curve observed when a dihydrochloride efletirizine immediate release tablet comprising said amount of efletirizine is dosed to same human subject at the same efletirizine dose.

Preferably the tablet according to the invention comprises an amount of pseudoephedrine which when dosed to a human subject gives a pseudoephedrine area under the pseudoephedrine plasma concentration versus time curve which is between 80 % and 125 % of the area under the pseudoephedrine plasma concentration versus time curve observed when a pseudoephedrine sustained release tablet comprising said amount of pseudoephedrine is dosed to same human subject.

Pseudoephedrine/efletirizine dosage forms of this invention provide pseudoephedrine and efletirizine blood or plasma levels which are equivalent to those resulting from dosing separate pseudoephedrine and efletirizine control formulation.

In the tablet according to the invention the particle size of the pseudoephedrine present is chosen such that it has a flow index less than 25. By "flow index" we understand the flowability index corresponding to the diameter of the smallest hole through which sample will pass three tests out of three (equipment from Hanson Research Corporation Chatsworth).

The particle size determination is carried out by means of airjet sifting under the following conditions : individual sieves according to ASTM E11, 10 g of substance, the equipment used is the Alpine airjet sieve, a low pressure is used, preferably 250 mm H₂O (between 100-300 mm H₂O), the sieving period is 5 minutes, and the auxiliary is 0.30 g antistatic per 10 g substance and preferably Aerosil R 972 (Degussa).

In the tablet according to the invention the particle size of the pseudoephedrine present is chosen such that it has an ability to settle of less than 30 ml. The ability to settle (V₁₀ - V₅₀₀) is measured according to Eur. Pharm. 2.9.15.

Preferably in the tablet according to the invention not more than 10% of the pseudoephedrine present therein has a particle size of less than 100 µm. More preferably the particle size of the pseudoephedrine is such that at least 95 % of the particles are less than 500 µm and not more than 15% are less than 106 µm.

The best results have been obtained with a tablet wherein the pseudoephedrine is crystalline.

The tablet according to a preferred embodiment of the invention comprises, as hydrophilic polymer, a methyl cellulose ether derivative and preferably a substituted hydroxylated methyl cellulose.

The viscosity of the methyl cellulose ether derivative is measured according to Eur. Pharm. described method in cellulose derivatives monographs or according to USP method n° <911 > .

The best results have been obtained with the product sold under the trademark Methocel K15 MCR, which is an hydroxypropylmethylcellulose (methoxyl : 19 - 24 %, hydroxypropyl : 7 - 12 %), chlorides : max 0.5 %; having an apparent viscosity of 11000 to 21000 mPa (=cP) and a particle size : min 90 % < 100 mesh.

Preferably the ratio of hydroxypropylmethylcellulose (HPMC) to the pseudoephedrine is between 0.5 to 2 by weight.

In the tablet according to a preferred embodiment of the invention the efletirizine containing segment also contains a disintegrant, preferably in the range less than 5 % by weight of efletirizine segment and most preferably in the range of 1 to 5 %. Examples of suitable disintegrant are sodium starch glycolate, sodium crosscarmelose (cross-linked carboxy methyl cellulose), polyvinylpyrrolidone derivatives, crospovidone (trademark Polyplasdone XL, PLP XL). The best results have been obtained with a disintegrant being a cross-linked carboxy methyl cellulose.

In a preferred embodiment of the tablet the efletirizine segment contains excipients including a polyhydroxyl compound having a molecular weight of less than 400. Preferably the polyhydroxyl compound is a sugar. Most preferably the sugar is lactose.

The tablet of the invention is a bi-layer tablet, the efletirizine segment being a layer and the pseudoephedrine segment being a layer. Preferably the weight ratio of the pseudoephedrine layer to the efletirizine layer is between 0.25 to 10, and most preferably between 2 and 6.

In the preferred embodiment the outer face of each of the two layers has a different shape. Preferably the tablet has a first face which is the pseudoephedrine layer, having multiple radii of curvature, and most preferably three. Preferably the tablet has a second face which is the efletirizine layer, having a single radius of curvature. Radius of curvature is defined in American Pharmaceutical Association (Tableting Specification Manual, 4th edition, 2215 Constitution Avenue, NW, Washington, DC 20037-2985, pp 45 and 46); cup radius is a single arc generated from the tablet's centerline (midpoint) across the tablet's diameter, minor axis or major axis; the cup radius forms the cup's profile; cup is the depression, or concavity, at the end of a punch tip; Major axis : length of a shaped tablet, minor axis is width of a shaped tablet.

A tablet may comprise an additional coating layer. In an alternative the coating layer can act as a taste masking agent. Examples of suitable taste masking agents are cellulose derivatives (methyl-, carboxymethyl- hydroxymethyl-, hydroxy ethyl-, hydroxymethylpropyl, cellulose) vinyl derivatives (polyvinyl alcohol, polyvinyl acetate), acrylic and methacrylic derivatives (Eudragits^{®}), maleic copolymers, polyoxyethylene glycols, natural resins (zeine, gums).

A tablet may also contain some pharmaceutically acceptable fillers as excipients. Examples of suitable fillers are starch and derivatives, lactose, mannitol, sucrose, glucose, sorbitol, calcium phosphates, maltodextrines, polyvinylpyrrolidone, polyethylene glycols, microcrystalline cellulose, organic acids.

In a preferred embodiment of the invention the tablet is packaged in a moisture and oxygen protective packaging material.

In a tablet according to a preferred embodiment of the invention, the pseudoephedrine segment comprises at least one excipient, selected from inert matrices, hydrophilic matrices, lipid matrices, mixtures of inert matrices and of lipid matrices, mixtures of hydrophilic matrices and of lipid matrices, mixtures of hydrophilic matrices and of inert matrices.

The tablets according to a preferred embodiment of the present invention comprise matrix excipients chosen from inert, hydrophilic and lipophilic matrices.

Examples of inert matrices which can be used according to the present invention are: polyvinyl chloride, polyethylene, vinyl acetate/vinyl chloride copolymers, polymethylmethacrylates, polyamides, silicones, ethyl cellulose, polystyrene and the like.

Examples of hydrophilic matrices which can be used according to the present invention are cellulose derivatives (hydroxypropyl methylcellulose, hydroxyethyl cellulose, hydroxypropyl cellulose, methylcellulose and the like), noncellulose polysaccharides (galactomannans, guar gum, carob gum, gum arabic, sterculia gum, agar, alginates and the like) and acrylic acid polymers (carbopols 934P and 974P and the like). The hydrophilic matrices preferably used according to the present invention are hydroxypropyl methyl celluloses, such as compounds sold under the trademark METHOCEL K or E.

Examples of lipid matrices which can be used according to the present invention are glycerides (mono-, di- or triglycerides: stearin, palmitin, laurin, myristin, hydrogenated castor or cottonseed oils, precirol and the like), fatty acids and alcohols (stearic acid, palmitic acid, lauric acid; stearyl alcohol, cetyl alcohol, cetostearyl alcohols, and the like), fatty acid esters (monostearates of propylene glycol and of sucrose, sucrose distearate and the like) and waxes (white wax, cachalot wax and the like).

In addition to the above-mentioned components, the tablet according to the present invention may also contain other excipients such as diluents (example: Emcompress, lactose and the like), binders (Avicel, starches, polyvinylpyrrolidone and the like), disintegrants (starches and modified starches, cellulose derivatives, alginic derivatives, pectins and the like), lubricants (talc, magnesium stearate, colloidal silica and the like), taste-masking agents (α-cyclodextrin, β-cyclodextrin, γ-cyclodextrin and their alkylated derivatives), flavourings or colourings as well as coating agents (example: cellulose derivatives, methacrylic resins, polyvinyl chloride, nylons and the like).

For implementing the method of treatment of the invention the tablet hereinabove described should contain an effective amount of efletirizine and pseudoephedrine. An effective amount can be readily determined by the use of conventional techniques and by observing results obtained under analogous circumstance. In determining the effective amount, a number of factors are considered including, but not limited to: the species of patient; its size, age, and general health; the specific disease involved; the degree of or involvement or the severity of the disease; the response of the individual patient; the particular compound administered; the mode of administration; the bioavailability characteristics of the preparation administered; the dose regimen selected; and the use of concomitant medication.

Additionally, the respective proportions of efletirizine and pseudoephedrine in the tablet should preferably be such that the said tablet comprises about 0.25 to about 2.5 percent by weight of efletirizine and about 10 to about 45 percent by weight of pseudoephedrine.

A tablet according to the invention can be administered to a patient in any form or mode which makes the tablet bioavailable in effective amounts, namely the oral route. One skilled in the art of preparing formulations can readily select the proper form and mode of administration depending upon the particular characteristics of the disease state to be treated, the stage of the disease, and other relevant circumstances.

The tablets of the invention can comprise at least one pharmaceutically acceptable excipient, the proportion and nature of which are determined by the solubility and chemical properties of the tablet selected, the chosen route of administration, and standard pharmaceutical practice.

More particularly, the present invention contemplates pharmaceutical compositions consisting essentially of a therapeutically effective amount of the above-described active compounds in association with one or more pharmaceutically acceptable excipients.

The excipient material may be a solid or semi-solid material which can serve as a vehicle or medium for the active ingredient. Suitable excipient materials are well known in the art. The pharmaceutical tablets of the invention may be adapted for oral use and may be administered to the patient in the form of tablets, or capsules.

The excipient material should be suitably selected with respect to the intended form of administration, and consistent with conventional pharmaceutical practice. For instance, for oral administration in the form of tablets or capsules, the therapeutically active drug components may be combined with any oral non-toxic pharmaceutically acceptable inert excipient such as lactose or starch. Optionally, the pharmaceutical tablet of the invention also contain a binder such as microcrystalline cellulose, gum tragacanth or gelatine, a disintegrating agent such as alginic acid, a lubricant such as magnesium stearate, a glidant such as colloidal silicon dioxide, a sweetening agent such as sucrose or saccharin, a coloring agent or a flavouring agent such as peppermint or methyl salicylate

Because of their easy administration, tablets represent the most advantageous oral dosage unit form. If desired, tablets may be coated by standard aqueous or nonaqueous techniques with sugar, shellac or other coating agents, for exemple enteric coating agents. Desirably, each tablet or capsule contains from about 15 mg to about 300 mg of the active ingredients.

A tablet according to the invention can be prepared according to various methods known to persons skilled in the art.

The present invention concerns also the use of a tablet described, for the manufacture of a medicament for preventing or treating disorders or conditions associated with rhinitis, cold, flu, cold-like and flu-like symptoms and allergic rhinitis, relief of nasal congestion, seasonal rhinitis, sneezing, rhinorrhea, nasal and ocular pruritus, redness of the eyes, tearing, sneezing.

The present invention concerns also a method for preventing or treating in humans and mammals disorders or conditions associated with rhinitis, cold, flu, cold-like and flu-like symptoms and allergic rhinitis, relief of nasal congestion, seasonal rhinitis, sneezing, rhinorrhea, nasal and ocular pruritus, redness of the eyes, tearing, sneezing.

The invention is further defined by reference to the following examples describing in detail the tablets of the present invention, as well as their utility.

### Example 1. Composition of the pseudoephedrine slow release segment of the bi-layer tablets.

A phase one, opened, randomised pilot study compared the oral bioavailability of experimental 120 mg sustained release segment pseudoephedrine formulations (table 1).

**Table 1. Composition of tablets A and B.**

| mg/tablet | | |
|---|---|---|
| Components | A | B |
| Pseudoephedrine. HCl | 120 | 120 |
| HPMC (a) | - | 120 |
| HPMC (b) | 200 | - |
| Microcrystalline cellulose | 74 | 55.5 |
| Colloidal silicon dioxide | 2 | 1.5 |
| Magnesium stearate | 4 | 3 |

| | | |
|---|---|---|
| HPMC (a) represents a compound hydroxypropyl methylcellulose having an apparent viscosity of 11250 to 21000 mPA (=cP (centipoises)), as defined in USP monograph hydroxypropyl methylcellulose. HPMC (b) represents a compound hydroxypropyl methylcellulose having an apparent viscosity of 80000 to 120000 mPa (=cP). | | |

The objective was to compare the oral bioavailability of the experimental sustained release formulations and an immediate release reference tablet (60 mg) given twice a day in 8 healthy male subjects.

The main pharmacokinetic parameters are listed in table 2.

**Table 2. Main pharmacokinetic parameters after oral administration of 120 mg of pseudoephedrine in 8 healthy volunteers Treatment**

| | Reference | A | B |
|---|---|---|---|
| Cₘₐₓ (ng/mL) | 391 | 259 | 295 |
| tₘₐₓ (h) | 1.5 | 5 | 5 |
| AUC (ng.h/mL) | 3877 | 3943 | 4249 |

The two experimental formulations (A and B), which showed a clear slow release profile, were bioequivalent to the reference formulation.

The B formulation was chosen for further development as pseudoephedrine layer given its longer plateau time in the curve compared to formulation A.

Example 2. Dissolution profile's pH dependence for tablet B segment.

Dissolution profile of pseudoephedrine is assessed at various pHs (water, HCl 0.1 N, pH 4.5, 6.8 and 7.5, USP 24 Apparatus 1, 100 rpm, 37°C). Results are expressed in table 3.

**Table 3. In vitro dissolution data of tablet B segment at various pHs.**

| Time (h) | Water | HCl 0.1 N | pH 4.5 | pH 6.8 | pH 7.5 |
|---|---|---|---|---|---|
| 0 | - | - | - | - | - |
| 1 | 44.1 | 39.4 | 39.6 | 40.4 | 41.4 |
| 2 | 62.1 | 57.0 | 58.0 | 58.2 | 59.4 |
| 3 | 74.9 | 68.5 | 70.1 | 70.8 | 70.5 |
| 4 | 84.0 | 77.9 | 79.0 | 79.6 | 79.2 |
| 6 | 92.7 | 89.3 | 92.3 | 90.8 | 90.6 |
| 8 | 97.8 | 97.3 | 96.7 | 96.7 | 96.2 |
| 12 | - | 105.3 | 101.2 | 100.9 | 99.9 |

The results show a pH-independent in vitro dissolution.

### Example 3. Composition efletirizine.HCl/pseudoephedrine.HCl 10 mg/ 120 mg bi-layer tablet.

Coated efletirizine.HCl/pseudoephedrine.HCl bi-layer tablets were prepared.

The particle size of the pseudoephedrine is such that at least 95% of the particles are less than 500 µm and not more than 15% are less than 106 µm.

The formulation of these tablets is presented in table 4.

**Table 4. Composition of 10 mg /120 mg film coated efletirizine.HCl/pseudoephedrine.HCl tablets**

| | | mg/tablet |
|---|---|---|
| Core's first layer : | | |
| | Pseudoephedrine. HCl | 120 |
| | HPMC (a) | 120 |
| | Microcrystalline cellulose | 57 |
| | Colloidal silicon dioxide | 1.5 |
| | Magnesium stearate | 1.5 |
| Core's second layer : | | |
| | Efletirizine. HCl | 10.00 |
| | Lactose monohydrate | 91.20 |
| | Microcrystalline cellulose | 50.00 |
| | Croscarmellose sodium | 6.40 |
| | Colloidal silicon dioxide | 0.80 |
| | Magnesium stearate | 1.60 |
| Coating material: | Opadry white | 13.80 |

The product Opadry white is a combination of polymers for the aqueous film coating (hydroxypropylmethylcellulose, titanium dioxide, polyethylene glycol 400).

The components of each core layer are mixed separately and then compressed in a bi-layer rotary tablet press. Then the tablets are coated with Opadry.

The tablet has a first face, which is the pseudoephedrine layer, having multiple radii of curvature. The tablet has a second face, which is the efletirizine layer, having a single radius of curvature.

The interfacial surface area of the pseudoephedrine segment and efletirizine segment is about 95 mm². The diameter of the tablet is about 11 mm.

The tablet is packaged in a moisture and oxygen protective packaging material.

## Claims

1. A bi-layer tablet comprising at least two distinct segments, one segment of which comprises as active ingredient predominantly efletirizine and a second segment of which comprises as active ingredient predominantly pseudoephedrine, wherein the tablet is substantially free of impurities formed by reaction of efletirizine with pseudoephedrine, and with the proviso that the tablet comprises less than 5 % by weight, relative to the total weight of the pseudoephedrine segment, of an alkalinizing agent.

2. A tablet according to claim 1 wherein the pseudoephedrine segment is substantially free of efletirizine.

3. A tablet according to claim 1 wherein the efletirizine segment is substantially free of pseudoephedrine.

4. A tablet according to any one of the preceding claims wherein the interfacial surface area of the pseudoephedrine segment and efletirizine segment is less than 180 mm2_{.}

5. A tablet according to any one of the preceding claims wherein the tablet further comprises a barrier segment wherein said barrier segment separates the efletirizine segment and the pseudoephedrine segment.

6. A tablet according to any one of the preceding claims wherein the pseudoephedrine segment comprises less than 5 % by weight, relative to the total weight of the pseudoephedrine segment, of an alkalinizing agent.

7. A tablet according to any one of the preceding claims wherein the tablet comprises a plurality of pseudoephedrine segments.

8. A tablet according to any one of the preceding claims wherein the pseudoephedrine segment contains inert pharmaceutical excipients in an amount of 0.75 to 4.5 times that of the pseudoephedrine itself by weight.

9. A tablet according to any one of the preceding claims wherein the efletirizine segment contains inert pharmaceutical excipients in an amount of 5 to 30 times that of the efletirizine itself by weight.

10. A tablet according to any one of the preceding claims wherein the ratio of the total amount of inert pharmaceutical excipients present to the total aggregate amount of all active ingredients is between 1.2 and 6 by weight.

11. A tablet according to any one of the preceding claims wherein the weight ratio of pseudoephedrine to efletirizine is between 2 and 40.

12. A tablet according to claim 11 wherein the weight ratio of pseudoephedrine to efletirizine is about 12.

13. A tablet according to any one of the preceding claims wherein the pseudoephedrine segment comprises between about 10 and 265 mg of pseudoephedrine and the efletirizine segment comprises between about 3 and 70 mg of efletirizine.

14. A tablet according to any one of the preceding claims wherein the pseudoephedrine segment is in a slow release form.

15. A tablet according to any one of the preceding claims wherein the efletirizine is in an immediate release form.

16. A tablet according to any one of the preceding claims wherein the tablet weight is between 200 to 800 mg.

17. A tablet according to any one of the preceding claims wherein the tablet comprises an amount of efletirizine which when dosed to a human subject gives a efletrizine area under the plasma efletirizine concentration versus time curve which is between 80 % and 125 % of the area under the plasma efletirizine concentration versus time curve observed when a dihydrochloride efletirizine immediate release tablet comprising said amount of efletirizine is dosed to same human subject at the same efletirizine dose,

18. A tablet according to any one of the preceding claims wherein the tablet comprises an amount of pseudoephedrine which when dosed to a human subject gives a pseudoephedrine area under the pseudoephedrine plasma concentration versus time curve which is between 80 % and 125 % of the area under the plasma pseudoephedrine concentration versus time curve observed when a pseudoephedrine sustained release tablet comprising said amount of pseudoephedrine is dosed to same human subject.

19. A tablet according to any one of the preceding claims wherein the particle size of the pseudoephedrine present is chosen such that it has a flow index less than 25.

20. A tablet according to any one of the preceding claims wherein the particle size of the pseudoephedrine present is chosen such that it has an ability to settle of less than 30

21. A tablet according to any one of the preceding claims wherein not more than 10% of the pseudoephedrine present therein has a particle size of less than 100 µm.

22. A tablet according to claim 20 or 21 wherein the particle size of the pseudoephedrine is such that at least 95% of the particles are less than 500 µm and not more than 15% are less than 106 µm.

23. A tablet according to any one of claims 20 to 22 wherein the pseudoephedrine is crystalline.

24. A tablet according to any one of the preceding claims wherein the pseudoephedrine containing segment also contains a methyl cellulose ether derivative having a viscosity of about 11,000 to 21,000 mPa.

25. A tablet according to claim 24 wherein the methyl cellulose ether derivative is a substituted hydroxylated methyl cellulose.

26. A tablet according to claim 24 wherein the methyl cellulose ether derivative is an hydroxypropylmethylcellulose.

27. A tablet according to claim 26 wherein the derivative is an hydroxypropylmethylcellulose (methoxy: 19 - 24 %, hydroxypropyl : 7 - 12 %), chlorides : max 0.5 %: having an apparent viscosity of 11250 to 21000 mPa and a particle size : min 90 % < 100 mesh.

28. A tablet according to any one of claims 24 to 27 wherein the ratio of hydroxypropylmethylcellulose (HPMC) to the pseudoephedrine is between 0.5 to 2 by weight.

29. A tablet according to any one of the preceding claims wherein the efletirizine containing segment also contains a disintegrant.

30. A tablet according to claim 29 wherein the efletirizine containing segment also contains a disintegrant in the range less than 5 % by weight of efletirizine segment.

31. A tablet according to claim 29 wherein the disintegrant is a cross-linked carboxy methyl cellulose.

32. A tablet according to any one of the preceding claims wherein the efletrizine segments contains excipients including a polyhydroxyl compound having a molecular weight of less than 400.

33. A tablet according to claim 32 wherein the polyhydroxyl compound is a sugar.

34. A tablet according to claim 33 wherein the sugar is lactose.

35. A tablet according to claim 34 wherein the weight ratio of the pseudoephedrine layer to the efletirizine layer is between 0.25 to 10.

36. A tablet according to claims 34 or 35 wherein the outer face of each of the two layers has a different shape.

37. A tablet according to claim 36 wherein the tablet has a first face which is the pseudoephedrine layer, having multiple radii of curvature

38. A tablet according to claim 36 wherein the tablet has a second face which is the efletirizfne layer, having a single radius of curvature.

39. A tablet according to anyone of the preceding claims which comprises an additional coating layer.

40. A tablet according to claim 39 wherein the coating layer can act as a taste mashing agent

41. A tablet according to anyone of the preceding claims wherein the tablet is packaged in a moisture protective packaging material.

42. A tablet according to anyone of the preceding claims wherein the tablet is packaged in an oxygen protective packaging material.

43. A tablet according to anyone of the preceding claims wherein the efletirizine segment comprises efletirizine dihydrochloride.

44. Use of a tablet according to anyone of the preceding claims, for the manufacture of a medicament for preventing or treating disorders or conditions associated with rhinitis, cold, flu, cold-like and flu-like symptoms, and allergic rhinitis, relief of nasal congestion, seasonal rhinitis, sneezing, rhinorrhea, nasal and ocular pruritus, redness of the eyes, tearing, sneezing.

## Patentansprüche

1. Doppelschichttablette, umfassend mindestens zwei gesonderte Segmente, wobei ein Segment davon als Wirkstoff überwiegend Efletirizin umfaßt und ein zweites Segment davon als Wirkstoff überwiegend Pseudoephedrin umfaßt, wobei die Tablette im wesentlichen frei von Verunreinigungen ist, die durch die Umsetzung von Efletirizin mit Pseudoephedrin gebildet werden, und mit der Maßgabe, daß die Tablette weniger als 5 Gew.-%, bezogen auf das Gesamtgewicht des Pseudoephedrinsegments, eines Alkalisierungsmittels umfaßt.

2. Tablette nach Anspruch 1, wobei das Pseudoephedrinsegment im wesentlichen frei von Efletirizin ist.

3. Tablette nach Anspruch 1, wobei das Efletirizinsegment im wesentlichen frei von Pseudoephedrin ist.

4. Tablette nach einem der vorhergehenden Ansprüche, wobei die Grenzfläche des Pseudoephedrinsegments und Efletirizinsegments weniger als 180 mm² beträgt.

5. Tablette nach einem der vorhergehenden Ansprüche, wobei die Tablette ferner ein Sperrsegment umfaßt, wobei das Sperrsegment das Efletirizinsegment und das Pseudoephedrinsegment trennt.

6. Tablette nach einem der vorhergehenden Ansprüche, wobei das Pseudoephedrinsegment weniger als 5 Gew.-%, bezogen auf das Gesamtgewicht des Pseudoephedrinsegments, eines Alkalisierungsmittels umfaßt.

7. Tablette nach einem der vorhergehenden Ansprüche, wobei die Tablette eine Vielzahl von Pseudoephedrinsegmenten umfaßt.

8. Tablette nach einem der vorhergehenden Ansprüche, wobei das Pseudoephedrinsegment inerte pharmazeutische Hilfsstoffe in einer Menge des 0,75- bis 4,5 fachen von der des Pseudoephedrins selbst, bezogen auf das Gewicht, enthält.

9. Tablette nach einem der vorhergehenden Ansprüche, wobei das Efletirizinsegment inerte pharmazeutische Hilfsstoffe in einer Menge des 5- bis 30 fachen von der des Efletirizins selbst, bezogen auf das Gewicht, enthält.

10. Tablette nach einem der vorhergehenden Ansprüche, wobei das Verhältnis der Gesamtmenge der vorliegenden inerten pharmazeutischen Hilfsstoffe zu der Gesamtaggregatmenge von allen Wirkstoffen zwischen 1,2 und 6, bezogen auf das Gewicht, liegt.

11. Tablette nach einem der vorhergehenden Ansprüche, wobei das Gewichtsverhältnis von Pseudoephedrin zu Efletirizin zwischen 2 und 40 liegt.

12. Tablette nach Anspruch 11, wobei das Gewichtsverhältnis von Pseudoephedrin zu Efletirizin etwa 12 beträgt.

13. Tablette nach einem der vorhergehenden Ansprüche, wobei das Pseudoephedrinsegment zwischen etwa 10 und 265 mg Pseudoephedrin umfaßt und das Efletirizinsegment zwischen etwa 3 und 70 mg Efletirizin umfaßt.

14. Tablette nach einem der vorhergehenden Ansprüche, wobei das Pseudoephedrinsegment in einer Form mit langsamer Freisetzung vorliegt.

15. Tablette nach einem der vorhergehenden Ansprüche, wobei das Efletirizin in einer Form mit sofortiger Freisetzung vorliegt.

16. Tablette nach einem der vorhergehenden Ansprüche, wobei das Tablettengewicht zwischen 200 und 800 mg liegt.

17. Tablette nach einem der vorhergehenden Ansprüche, wobei die Tablette eine Menge Efletirizin umfaßt, die, wenn an einen menschlichen Patienten verabreicht, eine Efletirizinfläche unter der Kurve der Plasma-Efletirizin-Konzentration gegen die Zeit ergibt, die zwischen 80 % und 125 % der Fläche unter der Kurve der Plasma-Efletirizin-Konzentration gegen die Zeit liegt, die beobachtet wird, wenn eine Dihydrochlorid-Efletirizin-Tablette mit sofortiger Freisetzung, die diese Menge an Efletirizin umfaßt, an denselben menschlichen Patienten bei derselben Efletirizindosis verabreicht wird.

18. Tablette nach einem der vorhergehenden Ansprüche, wobei die Tablette eine Menge an Pseudoephedrin umfaßt, die, wenn an einen menschlichen Patienten verabreicht, eine Pseudoephedrinfläche unter der Kurve der Pseudoephedrin-Plasma-Konzentration gegen die Zeit ergibt, die zwischen 80 % und 125 % der Fläche unter der Kurve der Plasma-Pseudoephedrin-Konzentration gegen die Zeit liegt, die beobachtet wird, wenn eine Pseudoephedrintablette mit anhaltender Freisetzung, die diese Menge an Pseudoephedrin umfaßt, an denselben menschlichen Patienten verabreicht wird.

19. Tablette nach einem der vorhergehenden Ansprüche, wobei die Teilchengröße des vorhandenen Pseudoephedrins so gewählt ist, daß es einen Fließexponenten von weniger als 25 aufweist.

20. Tablette nach einem der vorhergehenden Ansprüche, wobei die Teilchengröße des vorhandenen Pseudoephedrins so gewählt ist, daß es eine Absetzfähigkeit von weniger als 30 ml hat.

21. Tablette nach einem der vorhergehenden Ansprüche; wobei nicht mehr als 10 % des darin vorhandenen Pseudoephedrins eine Teilchengröße von weniger als 100 µm aufweisen.

22. Tablette nach Anspruch 20 oder 21, wobei die Teilchengröße des Pseudoephedrins so ist, daß mindestens 95 % der Teilchen kleiner als 500 µm sind und nicht mehr als 15 % kleiner als 106 µm sind.

23. Tablette nach einem der Ansprüche 20 bis 22, wobei das Pseudoephedrin kristallin ist.

24. Tablette nach einem der vorhergehenden Ansprüche, wobei das Pseudoephedrinenthaltende Segment ebenso ein Methylcelluloseetherderivat mit einer Viskosität von etwa 11.000 bis 21.000 mPa enthält.

25. Tablette nach Anspruch 24, wobei das Methylcelluloseetherderivat eine substituierte hydroxylierte Methylcellulose ist.

26. Tablette nach Anspruch 24, wobei das Methylcelluloseetherderivat eine Hydroxypropylmethylcellulose ist.

27. Tablette nach Anspruch 26, wobei das Derivat eine Hydroxypropylmethylcellulose ist (Methoxyl: 19 - 24 %, Hydroxypropyl: 7 - 12 %), Chloride: max. 0,5 %; mit einer scheinbaren Viskosität von 11250 bis 21000 mPa und einer Teilchengröße: min. 90 % < 100 Mesh.

28. Tablette nach einem der Ansprüche 24 bis 27, wobei das Verhältnis von Hydroxypropylmethylcellulose (HPMC) zu dem Pseudoephedrin zwischen 0,5 und 2, bezogen auf das Gewicht, liegt.

29. Tablette nach einem der vorhergehenden Ansprüche, wobei das Efletirizin-enthaltende Segment ebenso einen Lösungsvermittler enthält.

30. Tablette nach Anspruch 29, wobei das Efletirizin-enthaltende Segment ebenso einen Lösungsvermittler in dem Bereich von weniger als 5 Gew.-% des Efletirizinsegments enthält.

31. Tablette nach Anspruch 29, wobei der Lösungsvermittler eine vernetzte Carboxymethylcellulose ist.

32. Tablette nach einem der vorhergehenden Ansprüche, wobei das Efletirizinsegment Hilfsstoffe enthält, einschließlich einer Polyhydroxylverbindung mit einem Molekulargewicht von weniger als 400.

33. Tablette nach Anspruch 32, wobei die Polyhydroxylverbindung ein Zucker ist.

34. Tablette nach Anspruch 33, wobei der Zucker Lactose ist.

35. Tablette nach Anspruch 34, wobei das Gewichtsverhältnis der Pseudoephedrinschicht zu der Efletirizinschicht zwischen 0,25 und 10 liegt.

36. Tablette nach den Ansprüchen 34 oder 35, wobei die Außenfläche von jeder der zwei Schichten eine unterschiedliche Form aufweist.

37. Tablette nach Anspruch 36, wobei die Tablette eine erste Fläche, die die Pseudoephedrinschicht ist, mit mehreren Krümmungshalbmessern aufweist.

38. Tablette nach Anspruch 36, wobei die Tablette eine zweite Fläche, die die Efletirizinschicht ist, mit einem einzelnen Krümmungshalbmesser aufweist.

39. Tablette nach einem der vorhergehenden Ansprüche, die eine zusätzliche Beschichtungsschicht umfaßt.

40. Tablette nach Anspruch 39, wobei die Beschichtungsschicht als ein Geschmacksmaskierungsmittel fungieren kann.

41. Tablette nach einem der vorhergehenden Ansprüche, wobei die Tablette in einem vor Feuchtigkeit schützenden Verpackungsmaterial verpackt ist.

42. Tablette nach einem der vorhergehenden Ansprüche, wobei die Tablette in einem vor Sauerstoff schützenden Verpackungsmaterial verpackt ist.

43. Tablette nach einem der vorhergehenden Ansprüche, wobei das Efletirizinsegmet Efletirizindihydrochlorid umfaßt.

44. Verwendung einer Tablette nach einem der vorhergehenden Ansprüche zur Herstellung eines Medikaments zur Vorbeugung oder Behandlung von Störungen oder Zuständen, die mit Rhinitis, Erkältung, Grippe, erkältungsartigen und grippalen Symptomen und Rhinitis allergica, Linderung der Nasenschleimhautanschwellung, saisonaler Rhinitis, Niesen, Nasenfluß, nasalem und okularem Pruritus, Augenrötung, Tränenlaufen, Niesen in Verbindung stehen.

## Revendications

1. Comprimé bicouche comprenant au moins deux segments distincts, dont l'un des segments comprend comme principe actif d'une manière prépondérante de l'éflétirizine et dont un deuxième segment comprend comme principe actif d'une manière prépondérante de la pseudoéphédrine, le comprimé étant sensiblement exempt d'impuretés formées par réaction de l'éflétirizine sur la pseudoéphédrine, et sous réserve que le comprimé comprenne moins de 5 % en poids, par rapport au poids total du segment de pseudoéphédrine, d'un agent d'alcalinisation.

2. Comprimé suivant la revendication 1, dans lequel le segment de pseudoéphédrine est sensiblement exempt d'éflétirizine.

3. Comprimé suivant la revendication 1, dans lequel le segment d'éflétirizine est sensiblement exempt de pseudoéphédrine.

4. Comprimé suivant l'une quelconque des revendications précédentes, dans lequel la surface spécifique interfaciale du segment de pseudoéphédrine et du segment d'éflétirizine est inférieur à 180 mm².

5. Comprimé suivant l'une quelconque des revendications précédentes, dans lequel le comprimé comprend, en outre, un segment formant barrière qui sépare le segment d'éflétirizine et le segment de pseudoéphédrine.

6. Comprimé suivant l'une quelconque des revendications précédentes, dans lequel le segment de pseudoéphédrine comprend moins de 5 % en poids, par rapport au poids total du segment de pseudoéphédrine, d'un agent d'alcalinisation.

7. Comprimé suivant l'une quelconque des revendications précédentes, dans lequel le comprimé comprend une pluralité de segments de pseudoéphédrine.

8. Comprimé suivant l'une quelconque des revendications précédentes, dans lequel le segment de pseudoéphédrine contient des excipients pharmaceutiques inertes en une quantité représentant de 0,75 à 4,5 fois en poids celle de la pseudoéphédrine soi-même.

9. Comprimé suivant l'une quelconque des revendications précédentes, dans lequel le segment d'éflétirizine contient des excipients pharmaceutiques inertes en une quantité représentant de 5 à 30 fois en poids celle de l'éflétirizine soi-même.

10. Comprimé suivant l'une quelconque des revendications précédentes, dans lequel le rapport de la quantité totale des excipients pharmaceutiques inertes présents dans la quantité totale agrégée de tous les principes actifs est compris entre 1,2 et 6 en poids.

11. Comprimé suivant l'une quelconque des revendications précédentes, dans lequel le rapport en poids de la pseudoéphédrine à l'éflétirizine est compris entre 2 et 40.

12. Comprimé suivant la revendication 11, dans lequel le rapport en poids de la pseudoéphédrine à l'éflétirizine est d'environ 12.

13. Comprimé suivant l'une quelconque des revendications précédentes, dans lequel le segment de pseudoéphédrine comprend entre environ 10 et 265 mg de pseudoéphédrine et le segment d'éflétirizine comprend entre environ 3 et 70 mg d'éflétirizine.

14. Comprimé suivant l'une quelconque des revendications précédentes, dans lequel le segment de pseudoéphédrine est sous une forme à libération lente.

15. Comprimé suivant l'une quelconque des revendications précédentes, dans lequel l'éflétirizine est sous une forme à libération immédiate.

16. Comprimé suivant l'une quelconque des revendications précédentes, dans lequel le poids du comprimé est compris entre 200 et 800 mg.

17. Comprimé suivant l'une quelconque des revendications précédentes, dans lequel le comprimé comprend une quantité d'éflétirizine qui, lorsqu'elle est administrée de manière dosée à un sujet humain, donne une surface d'éflétirizine sous la courbe de la concentration d'éflétirizine dans le plasma en fonction du temps, qui est comprise entre 80 % et 125 % de la surface sous la courbe de la concentration de l'éflétirizine dans le plasma en fonction du temps, observée lorsque l'on administre au même sujet humain, à la même dose d'éflétirizine, un comprimé de dichlorhydrate d'éflétirizine à libération immédiate comprenant ladite quantité d'éflétirizine.

18. Comprimé suivant l'une quelconque des revendications précédentes, dans lequel le comprimé comprend une quantité de pseudoéphédrine qui, lorsqu'elle est administrée en une quantité dosée à un sujet humain, donne une surface de pseudoéphédrine sous la courbe de la concentration de pseudoéphédrine dans le plasma en fonction du temps, comprise entre 80 % et 125 % de la surface sous la courbe de la concentration de la pseudoéphédrine dans le plasma en fonction du temps, observée lorsque l'on administre au même sujet humain, un comprimé de pseudoéphédrine à libération prolongée comprenant ladite quantité de pseudoéphédrine.

19. Comprimé suivant l'une quelconque des revendications précédentes, dans lequel la dimension des particules de la pseudoéphédrine présente est choisie de manière à ce qu'elle ait un indice d'écoulement inférieur à 25.

20. Comprimé suivant l'une quelconque des revendications précédentes, dans lequel la dimension des particules de la pseudoéphédrine présente est choisie de manière à ce qu'elle ait une aptitude à décanter de moins de 30 ml.

21. Comprimé suivant l'une quelconque des revendications précédentes, dans lequel pas plus de 10 % de la pseudoéphédrine qui y est présente a une dimension de particule de moins de 100 µm.

22. Comprimé suivant la revendication 20 ou 21, dans lequel la dimension de particule de la pseudoéphédrine est telle qu'au moins 95 % des particules ont moins de 500 µm et pas plus de 15 % ont moins de 106 µm.

23. Comprimé suivant l'une quelconque des revendications 20 à 22, dans lequel la pseudoéphédrine est cristalline.

24. Comprimé suivant l'une quelconque des revendications précédentes, dans lequel le segment contenant de la pseudoéphédrine contient aussi un dérivé d'éther de méthyl cellulose ayant une viscosité d'environ 11 000 à 21 000 mPa.

25. Comprimé suivant la revendication 24, dans lequel le dérivé d'éther de méthyl cellulose est une méthyl cellulose hydroxylée substituée.

26. Comprimé suivant la revendication 24, dans lequel le dérivé d'éther de méthyl cellulose est une hydroxypropylméthylcellulose.

27. Comprimé suivant la revendication 26, dans lequel le dérivé est une hydroxypropylméthylcellulose (méthoxyle : 19 à 24 %, hydroxypropyle : 7 à 12 %), chlorures : max 0,5 % ; ayant une viscosité apparente de 11 250 à 21 000 mPa et une dimension de particule : min 90 % < 100 mesh.

28. Comprimé suivant l'une quelconque des revendications 24 à 27, dans lequel le rapport de l'hydroxypropylméthylcellulose (HPMC) à la pseudoéphédrine est compris entre 0,5 et 2 en poids.

29. Comprimé suivant l'une quelconque des revendications précédentes, dans lequel le segment contenant de l'éflétirizine contient aussi un agent de désintégration.

30. Comprimé suivant la revendication 29, dans lequel le segment contenant de l'éflétirizine contient aussi un agent de désintégration en une quantité représentant moins de 5 % du poids du segment d'éflétirizine.

31. Comprimé suivant la revendication 29, dans lequel l'agent de désintégration est une carboxy méthyl cellulose réticulée.

32. Comprimé suivant l'une quelconque des revendications précédentes, dans lequel le segment d'éflétirizine contient des excipients, y compris un composé polyhydroxylé ayant un poids moléculaire inférieur à 400.

33. Comprimé suivant la revendication 32, dans lequel le composé polyhydroxylé est un sucre.

34. Comprimé suivant la revendication 33, dans lequel le sucre est du lactose.

35. Comprimé suivant la revendication 34, dans lequel le rapport en poids de la couche de pseudoéphédrine à la couche d'éflétirizine est compris entre 0,25 et 10.

36. Comprimé suivant les revendications 34 ou 35, dans lequel la face extérieure de chacune des deux couches a une forme différente.

37. Comprimé suivant la revendication 36, dans lequel le comprimé a une première face qui est la couche de pseudoéphédrine ayant des rayons de courbure multiples.

38. Comprimé suivant la revendication 36, dans lequel le comprimé a une seconde face qui est la couche d'éflétirizine ayant un seul rayon de courbure.

39. Comprimé suivant l'une quelconque des revendications précédentes, qui comprend une couche supplémentaire d'enrobage.

40. Comprimé suivant la revendication 39, dans lequel la couche d'enrobage peut agir en tant qu'agent de masquage du goût.

41. Comprimé suivant l'une quelconque des revendications précédentes, dans lequel le comprimé est emballé dans une matière d'emballage de protection vis-à-vis de l'humidité.

42. Comprimé suivant l'une quelconque des revendications précédentes, dans lequel le comprimé est emballé dans une matière d'emballage de protection vis-à-vis de l'oxygène.

43. Comprimé suivant l'une quelconque des revendications précédentes, dans lequel le segment d'éflétirizine comprend du dichlorhydrate d'éflétirizine.

44. Utilisation d'un comprimé suivant l'une quelconque des revendications précédentes, pour la fabrication d'un médicament pour prévenir ou traiter des troubles ou des états associés à la rhinite, au rhume, à la grippe, à des symptômes de rhume et grippaux, et à la rhinite allergique, au soulagement de la congestion nasale, à la rhinite saisonnière, à l'éternuement, à la rhinorrhée, au prurit nasal et oculaire, à la rougeur des yeux, aux larmes et à l'éternuement.
